(19) Europäisches Patentamt
European Patent Office
Office européen des brevets

(11) Veröffentlichungsnummer: **0 166 297**
**B1**

# (12) EUROPÄISCHE PATENTSCHRIFT

(45) Veröffentlichungstag der Patentschrift: **31.08.88**

(51) Int. Cl.⁴: **C 07 D 251/70**, C 08 G 12/30, C 08 G 12/38

(21) Anmeldenummer: **85107179.5**

(22) Anmeldetag: **11.06.85**

## (54) Verfahren zur Herstellung von (2-Hydroxyethyl)melaminen.

(30) Priorität: **15.06.84 DE 3422218**

(43) Veröffentlichungstag der Anmeldung: **02.01.86 Patentblatt 86/1**

(45) Bekanntmachung des Hinweises auf die Patenterteilung: **31.08.88 Patentblatt 88/35**

(84) Benannte Vertragsstaaten: **AT BE CH DE FR GB IT LI NL SE**

(56) Entgegenhaltungen:
**EP-A-0 051 734**
**US-A-3 244 713**
**US-A-4 312 988**

(73) Patentinhaber: **BASF Aktiengesellschaft, Carl-Bosch- Strasse 38, D-6700 Ludwigshafen (DE)**

(72) Erfinder: **Ebel, Klaus, Dr., Ungsteiner Strasse 18, D-6700 Ludwigshafen (DE)**
Erfinder: **Reuther, Wolfgang, Dr., Am Pferchelhang 16, D-6900 Heidelberg (DE)**
Erfinder: **Weiss, Wolfram, Dr., Am Speyerweg 40, D-6704 Mutterstadt (DE)**
Erfinder: **Leigemann, Ludwig, Amsterdamer Strasse 16, D-6700 Ludwigshafen (DE)**

**EP 0 166 297 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von N,N',N''-Tris(2-hydroxethyl)melamin, gegebenenfalls in Mischung mit N-Mono- und N,N'-Bis(2-hydroxyethyl)melamin, durch Umsetzung von Melamin mit einem Überschuß an Ethanolamin sowie die Verwendung dieser (2-Hydroxyethyl)melamine als Modifizierungsmittel bei der Herstellung von Aminoplastharzen, die zur Oberflächenbeschichtung geeignet sind.

Es ist bekannt, die Aminogruppen des Melamins durch verschiedenartige Amine, wie Alkylamine, Arylamine oder auch Polyamine auszutauschen (US-A-2 328 961, US-A-2 393 755, US-A-2 467 523 und US-A-2 723 244).

Auch der Austausch mit Alkanolaminen, beispielsweise Ethanolamin oder Isopropanolamin wurde bereits untersucht (US-A-3 244 713 und EP-A-0 051 734).

So wird in der US-A-3 244 713 die Umsetzung von Melamin und Ethanolamin beschrieben, wobei Ethanolamin und Melamin in einem Molverhältnis von 5 : 1 in die Reaktion eingesetzt werden. Es zeigt sich allerdings, daß unter den dort genannten Reaktionsbedingungen nur unbefriedigende Ausbeuten an N,N',N''-Tris(2-hydroxyethyl)melamin erhalten werden. Gleichzeitig entstehen große Mengen an Nebenprodukten.

Aus der US-A-4 312 988 geht nun hervor, daß bei der Umsetzung von Melamin mit Ethanolamin in einer Konkurrenzreaktion unter Wasserabspaltung Isomelamine gebildet werden, die für die geringe Ausbeute an N,N',N''-Tris(2-hydroxyethyl)melamin verantwortlich sind. Beispielsweise wurden bei der Umsetzung von Ethanolamin mit Melamin im Molverhältnis 3,2 : 1 bei 82 % Umsatz 20 % an Isomelaminen, bei 95 % Umsatz bereits 50 % an Isomelaminen und bei 99 % Umsatz schließlich 100 % an Isomelaminen erhalten.

Es wurde daher vorgeschlagen, anstelle von Ethanolamin Isopropanolamin zu verwenden. Nach der Lehre der US-A-4 312 988 kann nämlich die Isomelaminbildung nur dann drastisch reduziert werden, wenn man das "geradkettige" Ethanolamin durch ein "verzweigtes" Isoalkanolamin ersetzt.

Aufgabe der vorliegenden Erfindung war es nun, ein neues Verfahren zur Herstellung von (2-Hydroxyethyl)melaminen, ausgehend von Melamin und Ethanolamin, bereitzustellen, in dem die Isomelaminbildung verhindert wird.

Entgegen der bestehenden Lehre wurde gefunden, daß die Herstellung von N,N',N''-Tris(2-hydroxyethyl)melamin, gegebenenfalls in Mischung mit N-Mono- und N,N'-Bis(2-hydroxyethyl)melamin, durch Umsetzung von Melamin mit Ethanolamin in Gegenwart von sauren Katalysatoren vorteilhaft gelingt, wenn man die Reaktion bei einer Temperatur von 120 bis 250°C und einem Überschuß an Ethanolamin durchführt und dabei ein Molverhältnis von freiem Ethanolamin : Summe aus freiem und umgesetztem Melamin von 4 : 1 während der gesamten Reaktionszeit nicht unterschreitet.

N,N',N''-Tris(2-hydroxyethyl)melamin, N,N'-Bis(2-hydroxyethyl)melamin und N'-Mono(2-hydroxyethyl)melamin gehorchen der allgemeinen Formel I

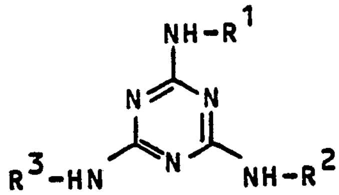

(I),

in der $R^1$, $R^2$ und $R^3$ jeweils folgende Bedeutung haben:

| | |
|---|---|
| $R^1 = R^2 = R^3 = -C_2H_4-OH$ | N,N',N''-Tris(2-hydroxyethyl)melamin |
| $R^1 = R^2 = -C_2H_4-OH$, $R^3 = H$ | N,N'-Bis(2-hydroxyethyl)melamin |
| $R^1 = C_2H_4OH$, $R^2 = R^3 = H$ | N-Mono(2-hydroxyethyl)melamin |

Mittels des erfindungsgemäßen Verfahrens erhält man nahezu isomelaminfreies N,N'-N''-Tris(2-hydroxyethyl)melamin, gegebenenfalls in Mischung mit der entsprechenden Mono- und Bisverbindung. Dadurch ist gewährleistet, daß die volle NH- und OH-Funktionalität dieser Produkte erhalten bleibt.

Das erfindungsgemäße Verfahren wird zweckmäßigerweise so durchgeführt, daß man ein Gemisch aus Melamin, Ethanolamin, saurem Katalysator und gegebenenfalls einem Lösungsmittel vorlegt und unter Rühren auf eine Temperatur von 120 bis 250°C, insbesondere von 150 bis 180°C erhitzt.

Man arbeitet im allgemeinen bei atmosphärischem Druck, um jedoch den oberen Temperaturbereich (180 bis 250°C) zu erreichen, muß ein Druck von 1 bis 15 bar eingehalten werden.

Weiterhin empfiehlt es sich, die Umsetzung in Gegenwart eines Schutzgases durchzuführen. Das Schutzgas wird dabei im allgemeinen über die Oberfläche der Reaktionsmischung geleitet. Geeignete Schutzgase sind z. B. Edelgase und insbesondere Stickstoff.

Als saure Katalysatoren kommen alle starken und mittelstarken Säuren in Betracht, z. B. Flußsäure, Salzsäure, Bromwasserstoffsäure, Schwefelsäure, Salpetersäure, Phosphorsäure, Amidosulfonsäure, Thiocyansäure, p-Toluolsulfonsäure oder Methansulfonsäure.

Die Säuren können entweder in freier Form oder als Melamin- oder Ethanolaminsalz zugegeben werden.

Weiterhin ist auch die Zugabe als Salz einer Base, die schwächer als Ethanolamin ist, möglich, beispielsweise als Ammoniumsalz.

Anstelle der genannten Protonsäure katalysieren auch Lewis-Säuren, wie Bortrifluorid, Aluminiumchlorid, Zinn(IV)chlorid, Antimon(V)fluorid oder Eisen(III)bromid die Reaktion.

Pro Mol Melamin werden 0,05 bis 3 Mol, vorzugsweise 0,1 bis 1 Mol Katalysator eingesetzt. Für den Fall, daß man die Protonsäure in Form ihres Melaminsalzes verwendet, muß die Melaminmenge aus dem Salz berücksichtigt werden. Das erforderliche Molverhältnis freies Ethanolamin : Summe aus freiem und umgesetztem Melamin von 4 : 1 darf nicht unterschritten werden. Mit zunehmender Katalysatormenge ist ein Anstieg der Reaktionsgeschwindigkeit zu beobachten.

Das erfindungsgemäße Verfahren wird vorzugsweise in Abwesenheit von Lösungsmitteln durchgeführt, jedoch ist es auch moglich in einem organischen Lösungsmittel zu arbeiten. Hierfür sind Polyole, z. B. Ethylenglykol, 1,2-Propylenglykol, Diethylenglykol oder Triethylenglykol geeignet.

Ein entscheidender Verfahrensparameter ist das Molverhältnis freies Ethanolamin : Summe aus freiem und umgesetztem Melamin, das während der gesamten Reaktionszeit den Wert 4 : 1 nicht unterschreiten darf. Als umgesetztes Melamin wird dabei die Summe an jeweils vorhandenem N-Mono-, N,N'-Bis- und N,N',N''-Tris(2-hydroxyethyl)melamin betrachtet.

Eine bevorzugte Ausführungsform des erfindungsgemäßen Verfahrens besteht darin, daß man während der gesamten Reaktionszeit ein Molverhältnis freies Ethanolamin: Summe aus freiem und umgesetztem Melamin von 6 : 1 nicht unterschreitet.

Pro Mol Melamin werden 6 bis 15 Mol, vorzugsweise 6 bis 10 Mol Ethanolamin eingesetzt.

Da eine Erhöhung des Ethanolaminüberschusses jedoch gleichzeitig auch eine Herabsetzung der Reaktionsgeschwindigkeit zur Folge hat, empfiehlt es sich, wenn man längere Reaktionszeiten vermeiden will, die Umsetzung mit einem geringeren Ethanolaminüberschuß zu beginnen und im Verlauf der Reaktion weiteres Ethanolamin hinzuzufügen.

Die Anfangsmenge an Ethanolamin beträgt dabei 6 bis 9 Mol, insbesondere 6 Mol, jeweils bezogen auf ein Mol Melamin. Die im Verlauf zugegebene Menge an Ethanolamin beträgt dabei bis zu 9 Mol, vorzugsweise bis zu 4 Mol, jeweils bezogen auf ein Mol Melamin. Die Zugabe kann dabei kontinuierlich oder diskontinuierlich in einem Zeitraum von 1 bis 10 Stunden erfolgen. Es ist jedoch auch möglich die jeweilige Ethanolaminmenge auf einmal zuzugeben.

Der Reaktionsverlauf kann mit analytischen Methoden, beispielsweise mittels Hochdruckflüssigkeitschromatographie (HPLC) verfolgt werden. Man kann die Reaktion bei jedem beliebigen Umsatzgrad abbrechen, wobei man Gemische aus N,N',N''Tris(2-hydroxyethyl)melamin, N-N'Bis(2-hydroxyethyl)melamin und N-Mono(2-hydroxyethyl)melamin erhält, die eine definierte, reproduzierbare Zusammensetzung aufweisen.

Zur Isolierung der Wertprodukte neutralisiert man die jeweilige Katalysatorsäure, indem man eine Base, z. B. Natriumhydryxid, Kaliumhydroxid, Natriumcarbonat, Kaliumcarbonat, Calciumcarbonat oder Bariumcarbonat in festem Zustand zur Reaktionsmischung gibt und die ausgefallenen Salze abtrennt.

Danach wird überschüssiges freies Ethanolamin bei vermindertem Druck (ca. 30 mbar) bei einer Temperatur von ca. 180°C abdestilliert, wobei der annähernd farblose Rückstand zu einem Harz erstarrt, das problemlos zerkleinert werden kann.

Wenn man die Reaktion bis zum vollständigen Umsatz (100 %) führt, gelingt die Herstellung von reinem, kristallinem N,N',N''-Tris(2-hydroxyethyl) melamin.

Dabei erfolgt die Aufarbeitung, wie oben beschrieben, ebenfalls durch Neutralisation, allerdings werden dann die ausgefallenen Salze nicht sofort abgetrennt, sondern der Ethanolaminüberschuß wird zunächst direkt abdestilliert. Der Rückstand wird dann in Butanol aufgenommen, vom anorganischen Salz abgetrennt und die butanolische Lösung mit Methylenchlorid versetzt. Beim Abkühlen fällt N,N',N''-Tris(2-hydroxyethyl)melamin in Form von farblosen Kristallen aus und wird abgesaugt.

Die genannten (2-Hydroxyethyl)melamine eignen sich hervorragend als Modifizierungsmittel bei der Herstellung von Aminoplastharzen, die zur Oberflächenbeschichtung geeignet sind (Tränkharze).

Aminoplastharze im allgemeinen Sinne sind harzartige Produkte und deren Lösungen, die durch Vorkondensation von Amino- oder Iminogruppen enthaltenden Verbindungen, den sogenannten Aminoplastbildnern, mit Carbonylverbindungen und gegebenenfalls Alkanolen entstehen und die unter Einfluß von hohen Temperaturen und/oder sauren Katalysatoren unter Vernetzung zu Makromolekülen weiterkondensieren können.

Ein wichtiger Anwendungsbereich der Aminoplaste, besonders der Melaminharze, ist die Herstellung von Schichtstoffen bzw. die Oberflächenbeschichtung von Plattenmaterialien. Dabei wird auf einen Träger, der seinerseits aus Kunststoff, bevorzugt aber aus einem Holzwerkstoff (Holzfaser- oder Holzspanplatte) besteht, eine Dekor- bzw. Schutzschicht dadurch aufgebracht, daß gemusterte oder einfarbige Papier- bzw. Gewebebahnen mit geeigneten Aminoplastharzen getränkt und mit einem bestimmten Restfeuchtegehalt auf die Platten des Werkstoffes auflaminiert (durch Druck aufgebracht) werden. Der Preßdruck kann dabei 1 bis 25 $N/mm^2$ und die Temperatur 120 bis 200°C betragen. Während des Preßvorganges härtet das Aminoplastharz thermisch aus, bildet eine Schutzschicht und verbindet die Papier- bzw. Gewebebahn mit der Platte des Werkstoffs. Die beschichteten Holzwerkstoffe und Schichtstoffe zeichnen sich durch gute chemische und physikalische Widerstandsfähigkeit aus.

Die bisher zum Tränken der für die Dekor- bzw. Schutzschicht vorgesehenen Papier- bzw. Gewebebahnen

benutzten Aminoplaste besitzen jedoch im ausgehärteten Zustand nur eine geringe Elastizität, weshalb mit diesen Harzen hergestellte Oberflächen zur Rißbildung neigen.

Es hat nicht an Versuchen gefehlt, die mangelnde Elastizität der Dekorschicht durch Zusätze zu den Tränkharzen zu beseitigen oder zu verringern. Insbesondere wurden als solche Modifizierungsmittel ε-Caprolactam, mehrwertige Alkohole, wie Sorbit und Zucker sowie aromatische Sulfonamide empfohlen. Bei derartigen Zusätzen wird jedoch die Wasserfestigkeit der ausgehärteten Harze ungenügend, wenn die Elastizität ausreichen soll.

Es wurde nun gefunden, daß das nach dem erfindungsgemäßen Verfahren hergestellte N,N',N"-Tris(2-hydroxyethyl)melamin, gegebenenfalls in Mischung mit N-Mono- und N,N'-Bis(2-hydroxyethyl)melamin vorteilhaft als Modifizierungsmittel bei der Herstellung von Aminoplastharzen, die zur Oberflächenbeschichtung geeignet sind, verwendet werden kann.

Die erfindungsgemäß zu verwendenden (2-Hydroxyethyl)melamine, vorzugsweise N,N',N"-Tris(2-hydroxyethyl)melamin, werden den Aminoplasten zweckmäßig in einer Menge von bis zu 15 Gew.%, vorzugsweise 0,5 bis 15 Gew.%, und insbesondere 5 bis 10 Gew.%, jeweils bezogen auf den Festkörpergehalt des Aminoplasten zugesetzt. Während geringere Mengen lediglich entsprechend geringere Wirkungen hervorrufen, können größere Mengen allmählich den Charakter des Aminoplastharzes selbst verändern. Auch muß bei größeren Anteilen unter Umständen noch Formaldehyd zur Härtung zugesetzt werden.

Als Aminoplastbildner kommen z. B. Harnstoff, Thioharnstoff, Dicyandiamid, Guanamine, wie Aceto- oder Benzoguanamin, insbesondere jedoch Melamin und als Carbonylverbindungen z. B. Acetaldehyd, aromatische Aldehyde, Ketone, insbesondere jedoch Formaldehyd, in Betracht. Auch Mischungen dieser Aminoplastbildner und/oder der genannten Carbonylverbindungen können zur Herstellung des Aminoplasten verwendet werden.

Melamin-Formaldehyd-Kondensationsprodukte und deren Mischkondensate oder Mischungen mit anderen Aminoplastbildnern und Formaldehyd sowie deren Veretherungsprodukte mit kurzkettigen Alkanolen haben sich zur Herstellung solcher Harze besonders bewährt. Vorzugsweise verwendet man Melamin-Formaldehyd-Kondensate, die 60 bis 100 Mol.-% Melamin als Aminoplastbildner und 60 bis 100 Mol.-% Formaldehyd als Carbonylverbindung enthalten. Dies bedeutet, daß jeweils noch 0 bis 40 Mol.-% anderer Aminoplastbildner bzw. Carbonylverbindungen vorhanden sein können.

Das Molverhältnis von Carbonylverbindungen zu Aminoplastbildern liegt, am Beispiel von Formaldehyd und Melamin dargestellt, im allgemeinen bei 1,4 : 1 bis 2,2 : 1, insbesondere 1,4 : 1 bis 2 : 1. Wenn außer Melamin andere Aminoplastbildner verwendet bzw. mitverwendet werden, ändert sich unter Umständen das Molverhältnis nach den bekannten Regeln geringfügig.

Die modifizierten Aminoplaste werden erhalten, indem man einen Aminoplastbildner mit einer Carbonylverbindung und gegebenenfalls mit üblichen modifizierten Mitteln in bekannter Weise in wäßriger Lösung kondensiert und vor, während oder nach der Kondensation das nach dem erfindungsgemäßen Verfahren hergestellte N,N',N"-Tris(2-hydroxyethyl)melamin, gegebenenfalls in Mischung mit der Mono- und Bis-Verbindung, in der oben genannten Menge zumischt. Bevorzugt ist der Zusatz während bzw. vor der Kondensation.

Den Aminoplasten können auch hydrolysierbare Salze schwacher bis starker Carbonsäuren, Sulfonsäuren oder Mineralsäuren, beispielsweise Diethanolaminacetat, Ethanolaminhydrochlorid, Ethylendiaminacetat, Ammoniumrhodanid, Ammoniumlactat oder Ethylendiaminphosphat zugefügt werden, um die Hartung zu beschleunigen, ohne daß sich dadurch die Elastizität der Überzüge verschlechtert.

Bei der Herstellung der Harze können auch noch andere Modifizierungsmittel, wie ein- oder mehrwertige Alkohole, Zucker, Säureamide wie Dimethylformamid oder ε-Caprolactam, Salze der Amidosulfonsäure und aromatische Sulfonsäureamide zugefügt werden. Die Mittel können sich gegenseitig ergänzen oder teilweise vertreten. Die Kondensation der Harze wird in der Regel bis zu einer begrenzten Wasserverdünnbarkeit fortgeführt. In manchen Fällen, z. B. bei Zusatz von größeren Mengen von Salzen der Amidosulfonsäure können die erhaltenen Harze auch unbegrenzt wasserlöslich werden.

Vorprodukte zur Herstellung dekorativer Schichtstoffe oder dekorativ beschichteter Holzwerkstoffplatten werden in der Weise hergestellt, daß man Papier- oder Gewebebahnen mindestens in der Deckschicht mit der Aminoplastlösung imprägniert und auf einen bestimmten Restfeuchtegehalt bei Temperaturen von 110 bis 160°C trocknet. Die getränkten Papiere bzw. Gewebebahnen werden zusammen zur Herstellung von Schichtstoffen mit mehreren Lagen mit Phenolharz imprägnierter Kraftpapiere als Trägerschicht unter erhöhtem Druck und Temperatur zu einem Schichtstoff gepreßt. Die Preßtemperaturen betragen ca. 120 bis 160°C, der Preßdruck 8 bis 12 N/mm$^2$; die Preßzeit richtet sich nach der Stärke und - bei Etagen-Pressen - der Menge der pro Etage hergestellten Schichtstoffe und kann z. B. 10 bis 80 Minuten betragen. Vor dem Entfernen wird zweckmäßigerweise auf ca. 60 bis 70°C zurückgekühlt.

Zur Herstellung von beschichteten Holzwerkstoffen werden die getränkten Papier- bzw. Gewebebahnen auf die vorbereitete Holzwerkstoffplatte unter Drucken von meist 1,5 bis 2,5 N/mm$^2$ und Temperaturen von 120 bis 200°C aufgepreßt.

Aminoplaste, die das erfindungsgemäße Modifizierungsmittel enthalten, sind besonders geeignet für die Herstellung von Schichtstoffen und beschichteten Holzwerkstoffen. Es zeigt sich nämlich, daß die Rißbildung in der Oberfläche von Schichtstoffen und Holzwerkstoffen beseitigt bzw. die Elastizität der Oberfläche wesentlich erhöht werden kann, wenn die für die Dekor- bzw. Schutzschicht vorgesehene Papier- bzw. Gewebebahn mit einem erfindungsgemäß modifizierten Aminoplast getränkt und anschließend in an sich bekannter Weise auf die Holzwerkstoffplatte auflaminiert oder zu einem Schichtstoff verarbeitet wird.

Weiterhin sind die genannten (2-Hydroxyethyl)melamine wertvolle Zwischenprodukte, beispielsweise für die Synthese von Urethanen.

Die nachfolgenden Beispiele sollen die Erfindung näher erläutern.

A) Herstellung

**Beispiele 1 bis 5**

Die Beispiele 1 bis 5 wurden unter den selben Reaktionsbedingungen lediglich mit unterschiedlichem Ethanolaminüberschuß durchgeführt.

Beispiel 1 stellt ein Vergleichsbeispiel dar, das analog dem Beispiel 7 der US-A-3 244 713 durchgeführt wurde. Die als Katalysator verwendete Salzsäure wurde, wie dort beschrieben, als Melaminhydrochlorid eingesetzt.

In den Beispielen 2 bis 5 wurde jeweils Ammoniumchlorid als Katalysator verwendet.

Durchführung der Beispiele 1 bis 5:

Melamin, Katalysator und Ethanolamin wurden unter Rühren und Überleiten eines schwachen Stickstoffstroms in einem 200°C heißen Ölbad unter Rückfluß gerührt. Die Reaktion wurde durch stündliche Probenentnahme mittels HPLC und Titration verfolgt.

Die Einwaagen und die HPLC-Auswertung sind in der nachfolgenden Tabelle aufgeführt.

**Tabelle**

| | Beispiel 1 | | Beispiel 2 | | Beispiel 3 | | Beispiel 4 | | Beispiel 5 | |
|---|---|---|---|---|---|---|---|---|---|---|
| | Einwaage (g) | Molverhältnis | Einwaage (g) | Molverhältnis | Einwaage (g) | Molverhältnis | Einwaage (g) | Molverhältnis | Einwaage (g) | Molverhältnis |
| Melaminhydrochlorid | 48,8 | 1 | - | - | - | - | - | - | - | - |
| Melamin | - | - | 42,0 | 1 | 42,0 | 1 | 31,5 | 1 | 31,5 | 1 |
| Ethanolamin | 91,5 | 5 | 122,0 | 6 | 163,0 | 8 | 153,0 | 10 | 228,8 | 15 |
| Ammoniumchlorid | - | - | 17,8 | 1 | 17,8 | 1 | 13,4 | 1 | 13,4 | 1 |

| | HPLC-Auswertung (Flächen-%) | HPLC-Auswertung (Flächen-%) | HPLC-Auswertung (Flächen-%) | HPLC-Auswertung (Flächen-%) | HPLC-Auswertung (Flächen-%) |
|---|---|---|---|---|---|
| A) Tris-HEM* | 44 | 42 | 42 | 37 | 52 |
| Nebenprodukte | 22 | 9 | 3 | 2 | 0,3 |
| B) Bis-HEM** | 4 | 2 | 4 | 2 | 2 |
| Tris-HEM | 52 | 60 | 80 | 91 | 96 |
| Nebenprodukte | 41 | 38 | 16 | 7 | 2 |

*Tris-HEM = N,N',N''-Tris(2-hydroxyethyl)melamin

**Bis-HEM = N,N'-Bis(2-hydroxyethyl)melamin

Um einen Vergleich der Beispiele zu ermöglichen, wurde unter A) jeweils die Konzentration der Nebenprodukte bei etwa gleichem Anteil an Tris-Substitutionsprodukt (Tris-HEM) von etwa 40 Flächen-% aufgeführt.

Unter B) wurde die Nebenproduktmenge bei möglichst hohem Tris-HEM-Anteil verglichen.

**Beispiel 6**

Zu einer Mischung aus 1008 g (8,0 Mol) Melamin und 3910 g (64,0 Mol) Ethanolamin tropfte man unter Rühren 160 g (1,6 Mol) Schwefelsäure. Dann wurde die heterogene Reaktionsmasse unter Rühren zum Rückfluß erhitzt, wobei ein schwacher Stickstoffstrom übergeleitet wurde. Nach 25 bis 28 Stunden Reaktionszeit hatte die inzwischen klare farblose Lösung eine durch HPLC bestimmte Triazinzusammensetzung von etwa 11 Mol.-% Mono-, 49 Mol.-% Bis- und 40 Mol.-% Tris(2-hydroxyethyl)melamin, mit weniger als 2 % Isomelaminen, bezogen auf die Hydroxyethylmelamine. Zur Aufarbeitung wurde bei 100°C durch Zugabe von 128 g (3,2 Mol) festem Natriumhydroxid neutralisiert und die heiße Lösung durch eine G4-Fritte abgesaugt. Das Filtrat wurde

bei einer Temperatur von 180°C und einem Druck von 30 mbar vom Ethanolaminüberschuß befreit. Der Destillationsrückstand erstarrte beim Abkühlen zu einer fast farblosen harzartigen Masse (1650 g), die aus etwa 11 Mol.-% Mono-, 49 Mol.-% Bis- und 40 Mol.-% Tris(2-hydroxyethyl)melamin bestand und sich problemlos zerkleinern ließ. Das Produkt enthielt noch 3 % Ethanolamin, 0,2 % Natriumsulfat und maximal 3 % Isomelamin.

**Beispiel 7**

Man erhitzte, wie in Beispiel 6 beschrieben, 504 g (4 Mol) Melamin, 2440 g (40 Mol) Ethanolamin und 214 g (4 Mol) Ammoniumchlorid 27 Stunden unter Rückfluß. Danach wurde bei 100°C durch Zugabe von 160 g (4 Mol) festem Natriumhydroxid neutralisiert und das überschüssige Ethanolamin bei einer Temperatur von 150 bis 190°C und einem Druck von 30 mbar abdestilliert. Der fast farblose Rückstand wurde unter Erwärmen in 1000 ml n-Butanol gelöst, das ausgefallene Natriumchlorid heiß abgesaugt und das Filtrat bei 30 bis 40°C mit 300 ml Methylenchlorid versetzt. Beim Abkühlen kristallisierte das reine Produkt aus und wurde abgesaugt und getrocknet. Man erhielt 846 g (81 %) N,N',N''-Tris-(2-hydroxyethyl)melamin vom Schmp. 95°C.

B) Anwendung

**Beispiele 8 bis 10**

Dekorpapier mit einem Flächengewicht von 80 g/m$^2$ wurde mit einer wäßrigen Melamin-Formaldehyd-Harzlösung (Feststoffgehalt 55 Gew.-%) getränkt und 5 Minuten bei 160°C getrocknet. Der Harzauftrag betrug ca. 120 Gew.-%, bezogen auf das Rohpapiergewicht und die Restfeuchte ca. 6,5 Gew.-%, bezogen auf das behandelte Papier.

Das verwendete Melamin-Formaldehyd-Harz wies ein Molverhältnis Melamin : Formaldehyd von 1 : 1,7 auf und war mit einem sauren Härter auf einen Trübungspunkt von 4,5 Minuten bei 100°C eingestellt.

Die getränkten Papiere wurden mit einem Druck von 2 N/mm$^2$ unter folgenden Bedingungen auf Spanplatten, die eine Dicke von 16 mm aufwiesen, gepreßt:

a) 50 Sekunden bei 190°C, ohne Rückkühlung
b) 100 Sekunden bei 160°C, ohne Rückkühlung
c) 360 Sekunden bei 140°C, anschließend Rückkühlung auf 50°C unter Druck.

Die Abpreßungen erfolgten mit Zulagepolster. Die angegebenen Temperaturen beziehen sich auf die Temperatur der Presse.

Die Prüfergebnisse sind in der nachfolgenden Tabelle zusammengefaßt.

| | Beispiel 8 (Vergleich) | Beispiel 9 (Vergleich) | Beispiel 10 |
|---|---|---|---|
| Modifizierungsmittel | - | 1,4-Butandiol | N,N',N''-Tris(2--hydroxyethyl)-melamin |
| Menge, bezogen auf Harzlösung [Gew.-%] | - | 6 | 6 |
| Glanzgrad [Refraktometer-einheiten] | a) 60<br>b) 70<br>c) 90 | a) 25<br>b) 30<br>c) 70 | a) 40<br>b)50<br>c) 80 |
| Glanzänderung (nach 1 Std. Wasserdampf-einwirkung)* vorher | 90 | 70 | 80 |
| nachher | 85 | 35 | 70 |
| [Refraktometer-einheiten] | | | |
| Rißbildung (24 Std. bei 80° C)** | stark | keine | keine |

* vgl. DIN 53 799
** vgl. DIN 68 765, DIN 53 799

## Patentansprüche

1. Verfahren zur Herstellung von N,N',N''-Tris(2-hydroxyethyl)melamin, gegebenenfalls in Mischung mit N-Mono- und N,N'-Bis(2-hydroxyethyl)melamin, durch Umsetzung von Melamin mit Ethanolamin in Gegenwart von sauren Katalysatoren, dadurch gekennzeichnet, daß man die Reaktion bei einer Temperatur von 120 bis 250° C und einem Überschuß an Ethanolamin durchführt und dabei ein Molverhältnis von freiem Ethanolamin : Summe aus freiem und umgesetztem Melamin von 4 : 1 während der gesamten Reaktionszeit nicht unterschreitet.
2. Verfahren gemäß Anspruch 1, dadurch gekennzeichnet, daß man die Umsetzung bei einer Temperatur von 150 bis 180° C und bei atmosphärischem Druck durchführt.
3. Verfahren gemäß Anspruch 1, dadurch gekennzeichnet, daß man die Umsetzung in Abwesenheit eines Lösungsmittels durchführt.
4. Verfahren gemäß Anspruch 1, dadurch gekennzeichnet, daß man pro Mol Melamin 6 bis 15 Mol Ethanolamin verwendet.

## Claims

1. A process for the preparation of N,N',N''-tris-(2-hydroxyethyl)-melamine, if required as a mixture with N-mono- and N,N'-bis-(2-hydroxyethyl)-melamine reacting melamine with ethanolamine in the presence of an acidic catalyst, wherein the reaction is carried out at from 120 to 250° C using an excess of ethanolamine, and the molar ratio of free ethanolamine to the sum of free and converted melamine is not permitted to fall below 4: 1 during the entire reaction time.
2. A process as claimed in claim 1, wherein the reaction is carried out at from 150 to 180° C and under atmospheric pressure.
3. A process as claimed in claim 1, wherein the reaction is carried out in the absence of a solvent.
4. A process as claimed in claim 1, wherein from 6 to 15 moles of ethanolamine are used per mole of melamine.

## Revendications

1. Procédé de préparation de la N,N',N''-tris(2-hydroxyéthyl)mélamine, éventuellement en mélange à de la N-mono- et de la N,N'-bis(2-hydroxyéthyl)mélamine, par la réaction de la mélamine sur l'éthanolamine en

présence de catalyseurs acides, caractérisé en ce que l'on entreprend la réaction à une température de 120 à 250°C et avec un excès d'éthanolamine et on veille à ce que le rapport molaire de l'éthanolamine libre à la somme de la mélamine libre et convertie ne reste pas en dessous d'un rapport 4 : 1 au cours de la totalité de la période réactionnelle.

2. Procédé suivant la revendication 1, caractérisé en ce que l'on entreprend la reaction à une température de 150 à 180°C et à la pression atmosphérique.

3. Procédé suivant la revendication 1, caractérisé en ce que l'on entreprend la réaction en l'absence d'un solvant.

4. Procédé suivant la revendication 1, caractérisé en ce que l'on utilise de 6 à 15 moles d'éthanolamine par mole de mélamine.